# EUROPEAN PATENT APPLICATION

(11) **EP 1 698 343 A1**
(43) Date of publication of application: **06.09.2006**
(21) Application number: 05004658.0
(22) Date of filing: 03.03.2005
(51) Int. Cl.: A61K 36/00, A23L 1/30, A61P 3/10

(54) **Foodstuff produced with extract of peanut stem for regulating blood sugar**

(71) Applicant: CHEN, CHUEN-MI, Hsin-Ying City, Tainan County (TW)
(72) Inventor: CHEN, CHUEN-MI, Hsin-Ying City, Tainan County (TW)
(74) Representative: Sroka, Peter-Christian

(57) **Abstract**

A foodstuff is produced through extracting healthful and medicinal compositions contained in peanut stems. The foodstuff may be in the form of liquid, cream, powder, tablet or capsule for users to drink, eat, or swallow conveniently. The foodstuff produced with extract of peanut stem contains not only nutrient that is helpful in health, but also special medicinal compositions useful in regulating blood sugar and alleviating or even curing pains of patients having uric acid problems, and is therefore suitable for drinking or eating on a long-term basis.

## Description

### FIELD OF THE INVENTION

The present invention relates to a foodstuff produced mainly with extract of peanut stem. The foodstuff may be produced into different forms, such as liquid, cream, powder, capsule, and tablet, and has been found healthful and, particularly, useful in regulating blood sugar to alleviate the pains of patients having uric acid problems.

### BACKGROUND OF THE INVENTION

Seeds of peanut plant have been widely utilized by people either as food for eating directly or a material for producing edible oil through pressing or solvent extraction. However, leaves, stems, and roots of peanut plant are usually discarded as waste or used as an organic fertilizer and have never been studied for any possibly practical value thereof. As a matter of fact, the stem of peanut plant, which shall be referred to as peanut stem hereinafter for simplicity purpose, contains unique medicinal compositions and even healthful nutrient, and should not be treated as waste. It is therefore tried by the inventor to produce a foodstuff using extract of peanut stem.

### SUMMARY OF THE INVENTION

A primary object of the present invention is to provide a foodstuff produced with extract of peanut stem and helpful in regulating blood sugar.

To achieve the above and other objects, medicinal and healthful compositions contained in peanut stems are extracted and the extract is used to produce foodstuff in the form of liquid, cream, powder, tablet or capsule for users to drink, eat, or swallow conveniently.

The foodstuff of the present invention produced with extract of peanut stem contains not only nutrient that is helpful in health, but also special medicinal compositions useful in regulating blood sugar and alleviating or even curing pains of patients having uric acid problems, and is therefore suitable for drinking or eating on a long-term basis.

### BRIEF DESCRIPTION OF THE DRAWINGS

The structure and the technical means adopted by the present invention to achieve the above and other obj ects can be best understood by referring to the following detailed description of the preferred embodiments and the accompanying drawings, wherein
Fig. 1 is a flowchart showing steps for producing the foodstuff of the present invention in liquid form;
Fig. 2 is a flowchart showing steps for producing the foodstuff of the present invention in cream form;
Fig. 3 is a flowchart showing steps for producing the foodstuff of the present invention in powder form; and
Fig. 4 is a flowchart showing steps for producing the foodstuff of the present invention in capsule or tablet form.

### DETAILED DESCRIPTION OF THE PREFERRED EMBODIMENTS

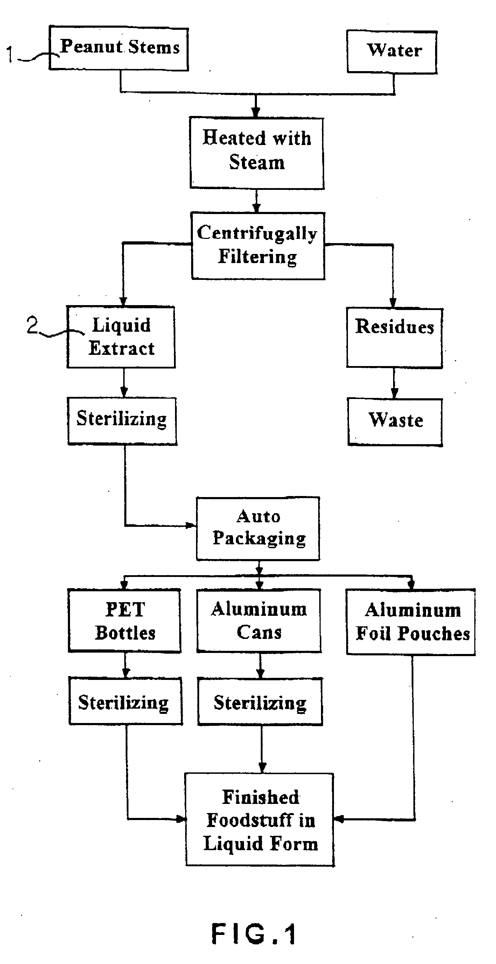

Please refer to Fig. 1 that is a flowchart showing steps for producing a foodstuff of the present invention in liquid form. As shown, peanut stems 1 are cleaned with water and then sun-dried under normal temperature or heated and baked at a temperature not exceeded 50°C. Thereafter, add 10 to 300 parts of water as solvent to one part of dried peanut stems 1, and slowly heat the water to a temperature within the range from 50°C to 100°C for 1 to 4 hours to completely extract the special compositions contained in the peanut stems 1. The peanut stems 1 are then centrifugally filtered to separate residues of peanut stems 1 from liquid extract 2 containing the special compositions. The residues are discarded, and the liquid extract 2 is subjected to instantaneous sterilization immediately before being packaged in aluminum foil pouches, aluminum cans, or PET bottles with an automatic packaging machine. The packaged liquid extract 2 is subjected to a second time sterilization, and a final product in liquid form is obtained for drinking directly.

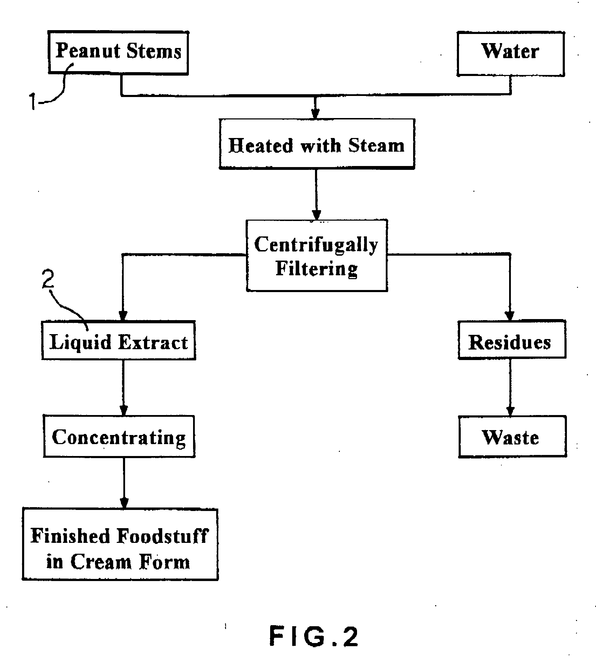

Alternatively, the liquid extract 2 obtained from the step of centrifugal filtering in Fig. 1 may be further processed to obtain a foodstuff in cream or paste form. Fig. 2 is a flowchart showing steps of producing a cream-form foodstuff of the present invention. As shown, the liquid extract 2 containing the special compositions of peanut stems 1 is subjected to concentration and dehydration under low temperature with vacuumizing or pressure-reducing apparatus until a desired cream or paste form is obtained.

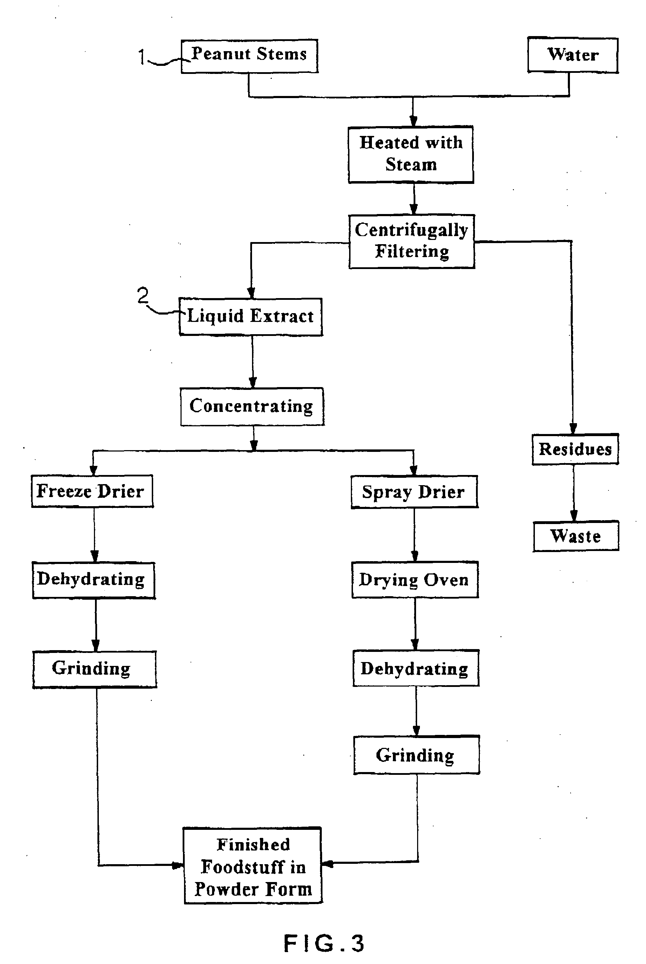

Alternatively, the cream or paste obtained from the liquid extract 2 in the step of concentration and dehydration in Fig. 2 may be further processed to obtain a foodstuff in powder form. Fig. 3 is a flowchart showing steps of producing a powder-form foodstuff of the present invention. As shown, when the cream or paste containing the special compositions of peanut stems 1 is further concentrated under a reduced pressure to a volume within the range from 1/5 to 1/10 of the initial liquid extract 2, it is sprayed with a spray drier into a drying oven, at where the sprayed cream or paste is quickly dehydrated into powder. Alternatively, the further concentrated cream or paste is subjected to freezing in a freeze drier under high vacuum and ultra-low temperature, and is thereby dehydrated into powder. The obtained powder is further ground to fine powder.

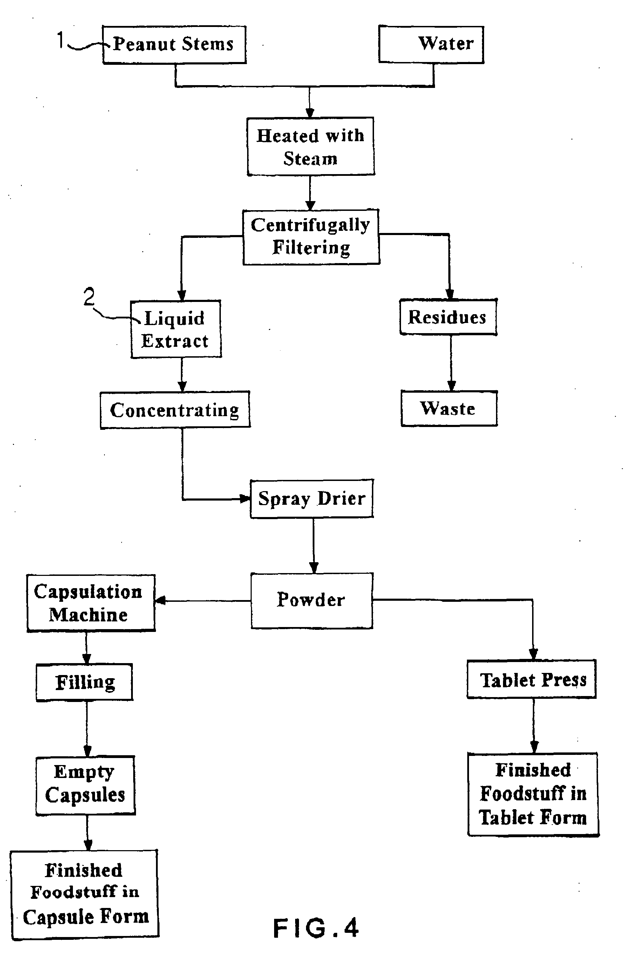

Alternatively, the fine powder obtained from the liquid extract 2 in the steps of spray drying, dehydrating, and grinding in Fig. 3 may be further processed to obtain a foodstuff in capsule or tablet form. Fig. 4 is a flowchart showing steps of producing a capsulated or tabletted foodstuff of the present invention. As shown, the obtained fine powder is compressed into tablets with a tablet press, or automatically packaged and enclosed in empty capsules with a capsulation machine.

The foodstuff of the present invention produced with extract of peanut stem has the following advantages:
1. It is useful in improving a user's health condition.
2. It provides obviously good effect in body health.
3. Experiments prove it has the functions of regulating blood sugar and alleviating and even curing the pains of patients having uric acid problems.
4. It maybe provided in various forms, including liquid, powder, cream, paste, tablet, and capsule, allowing users to take it conveniently.
5. It is produced from peanut stem that is otherwise discarded as waste, and therefore meets the requirement of environment protection and is industrially valuable.

## Claims

1. A foodstuff comprising extract of peanut stem to provide blood sugar regulating effect; said extract of peanut stem being obtained by having an amount of peanut stems washed clean, and sun-dried under normal temperature or heated and baked at a temperature not exceeded 50°C; adding 10 to 300 parts of water as solvent to one part of said dried peanut stems, and slowly heating said water to a temperature within the range from 50°C to 100°C for 1 to 4 hours to completely extract special medicinal compositions contained in said peanut stems; having said peanut stems centrifugally filtered to separate residues of said peanut stems from a liquid extract containing said special medicinal compositions; discarding said residues; and having said liquid extract instantaneously sterilized.

2. The foodstuff as claimed in claim 1, wherein said instantaneously sterilized liquid extract is packaged into separated containers with an automatic packaging machine and sterilized for a second time to allow a user to drink said foodstuff directly.

3. The foodstuff as claimed in claim 2, wherein said containers for said foodstuff are selected from the group consisting of aluminum foil pouches, aluminum can, and PET bottles.

4. The foodstuff as claimed in claim 1, wherein said liquid extract is further processed with vacuumizing or pressure-reducing apparatus under a low temperature, so that said liquid extract is concentrated and dehydrated into a form of cream.

5. The foodstuff as claimed in claim 4, wherein said cream obtained from said liquid extract is further concentrated under reduced pressure to a volume about 1/5 to 1/10 of said liquid extract, and is then sprayed into a drying oven with a spray drier to be quickly dehydrated into powder that is then ground into fine powder.

6. The foodstuff as claimed in claim 4, wherein said cream obtained from said liquid extract is further concentrated under reducedpressure to a volume about 1/5 to 1/10 of said liquid extract, and is then freeze-dried into powder under high vacuum and ultra-low temperature in a freeze drier, and said powder being further ground into fine powder.

7. The foodstuff as claimed in claims 5 and 6, wherein said fine powder is compressed into tablets with a tablet press.

8. The foodstuff as claimed in claims 5 and 6, wherein said fine powder is automatically packaged and enclosed in empty capsules with a capsulation machine.
